# EUROPÄISCHE PATENTANMELDUNG

(11) **EP 4 029 926 A1**
(43) Veröffentlichungstag der Anmeldung: **20.07.2022**
(21) Anmeldenummer: 21151735.4
(22) Anmeldetag: 15.01.2021
(51) Int. Cl.: C11D 7/26, C11D 7/50, C11D 11/00, C11D 17/00

(54) **ZWEISTUFIGE ABGASWÄSCHE**

(71) Anmelder: Evonik Operations GmbH, 45128 Essen (DE)
(72) Erfinder: Peitz, Stephan, 45739 Oer-Erkenschwick (DE); Stochniol, Guido, 45721 Haltern am See (DE); Oldemeyer, Martin, 45721 Haltern am See (DE); Böse, Matthias, 46348 Raesfeld (DE)
(74) Vertreter: Evonik Patent Association

(57) **Zusammenfassung**

Gegenstand der Erfindung ist ein Verfahren zur Entfernung von organischen Bestandteilen aus einem Gasstrom in einer zweistufigen Wäsche, bei der zunächst mit einem alkoholischen Waschmittel und anschließend mit einem wässrigen Waschmittel gewaschen wird. Die erhaltenen beladenen Waschmittel können in bestimmten (chemischen) Verfahren eingesetzt werden.

## Beschreibung

Die vorliegende Erfindung betrifft ein Verfahren zur Entfernung von organischen Bestandteilen aus einem Gasstrom in einer zweistufigen Wäsche, bei der zunächst mit einem alkoholischen Waschmittel und anschließend mit einem wässrigen Waschmittel gewaschen wird. Die erhaltenen beladenen Waschmittel können in bestimmten (chemischen) Verfahren eingesetzt werden.

In der chemischen Industrie fallen an vielen Stellen Abgasströme an, beispielsweise bei der Lagerung oder Abfüllung von Einsatzstoffen oder Produkten, wie Abgase aus der Tankbeatmung oder verdrängte Gase bei der Abfüllung von Tank- und Bahnkesselwagen. Diese Gasströme enthalten meistens organische Bestandteile, die aus den gelagerten oder abgefüllten Einsatzstoffen stammen können. Zur Einhaltung emissionsrechtlicher Vorgaben müssen diese Gasströme einer Abgaswäsche unterzogen werden, um die darin enthaltenen organischen Bestandteile auszuwaschen.

Die mit den organischen Bestandteilen beladenen alkoholischen und/oder wässrigen Waschmittel werden üblicherweise entsorgt. Eine solche Entsorgung ist nicht nur teuer, sondern erfordert einen vergleichsweise hohen Einsatz von Waschmittel. Eine Möglichkeit dies zu umgehen wäre eine Aufarbeitung der Waschmittel, was jedoch mit einem hohen technischen Aufwand verbunden sein kann.

Die Aufgabe der vorliegenden Erfindung war deshalb die Bereitstellung eines einfachen und kostengünstigen Verfahrens zur Entfernung von organischen Bestandteilen aus einem Gasstrom.

Die Aufgabe konnte durch das hier beschriebene Verfahren gelöst werden. Das erfindungsgemäße Verfahren ist ein Verfahren zur Entfernung von organischen Bestandteilen aus einem Gasstrom in einer zweistufigen Wäsche, wobei
der Gasstrom in einer ersten Wascheinheit mit einem flüssigen alkoholischen Waschmittel gewaschen wird, wodurch die organischen Bestandteile des zu reinigen Gasstroms zumindest teilweise in das Waschmittel übergehen und wodurch ein an organischen Bestandteilen reduzierter Gasstrom, der einen Teil des alkoholischen Waschmittels als alkoholische Bestandteile enthält, und ein mit organischen Bestandteilen beladenes alkoholisches Waschmittel erhalten wird; und
der an organischen Bestandteilen reduzierte Gasstrom in einer zweiten Wascheinheit mit einem wässrigen Waschmittel gewaschen wird, wodurch die alkoholischen Bestandteile zumindest teilweise in das Wachmittel übergehen und wodurch ein an organischen und alkoholischen Bestandteilen reduzierter Gasstrom und ein mit alkoholischen Bestandteilen beladenes wässriges Waschmittel erhalten wird;
dadurch gekennzeichnet, dass in beiden Wascheinheiten jeweils pro m³ zu reinigendem Gasstrom mindestens 0,7 kg Waschmittel und pro kg der zu entfernenden organischen Bestandteile mindestens 4 kg Waschmittel eingesetzt werden, und dass
das mit organischen Bestandteilen beladene alkoholische Waschmittel in ein (chemisches) Verfahren (zurück)geführt wird, in dem der Alkohol als Einsatzstoff verwendet wird, und das mit alkoholischen Bestandteilen beladene wässrige Waschmittel in ein (chemisches) Verfahren zurückgeführt wird, in dem das Wasser als Einsatzstoff oder Extraktionsmittel verwendet wird.

Der Begriff "(zurück)geführt" ist im Rahmen der vorliegenden Erfindung so zu verstehen, dass es sowohl den Umstand umfasst, dass das entsprechende Waschmittel aus einem (chemischen) Verfahren stammen kann, in das es zurückgeführt wird, also auch den Umstand umfasst, dass das entsprechende Waschmittel aus einem geeigneten Behälter frisch und damit ohne vorheriges Durchlaufen eines (chemischen) Verfahrens bereitgestellt wird, nach dem Waschschritt aber zu einem (chemischen) Verfahren geführt wird.

Der Begriff "(chemisches) Verfahren" meint sowohl Verfahren, bei dem das entsprechende Waschmittel als Edukt und damit Reaktionspartner eingesetzt wird, als auch Verfahren bei dem es als weiteres Wasch- oder Extraktionsmittel eingesetzt werden kann.

Bezogen auf das im ersten Waschschritt bzw. in der ersten Wascheinheit eingesetzte alkoholische Waschmittel, ist das (chemische) Verfahren, in das der Alkohol bzw. das alkoholische Waschmittel (zurück)geführt wird, eine Reaktion, bei der Alkohol als Edukt eingesetzt wird. Beispiele dafür sind die Herstellung von ATBE (Alkyl-tert.-butylether) oder die Alkoxycarbonylierung. Den vorherigen Ausführungen ist zu entnehmen, dass die Begriffe Alkohol und alkoholisches Waschmittel synonym verwendet werden. Für Methanol als möglichem alkoholischen Waschmittel sind als (chemische) Verfahren, bei denen Methanol als Edukt eingesetzt wird, eine Veretherung (z.B. der Synthese von MTBE = Methyl-tert.-butylether oder DME = Dimethylether), eine Methoxycarbonylierung oder eine Veresterung bevorzugt.

Bezogen auf das im zweiten Waschschritt bzw. in der zweiten Wascheinheit eingesetzte wässrige Waschmittel, ist das (chemische) Verfahren entweder ein Verfahren, bei dem Wasser Reaktionspartner ist oder als Wasch- oder Extraktionsmittel fungiert. Ein bevorzugtes (chemisches) Verfahren, bei dem das wässrige Waschmittel, insbesondere das Wasser, eingesetzt wird, sind die TBA-Synthese (TBA = tert.-Butanol), die Hydrolyse und die Hydroxycarbonylierung. Bevorzugte (chemische) Verfahren, bei denen das wässrige Waschmittel, insbesondere das Wasser, als Wasch- oder Extraktionsmittel eingesetzt wird, sind die ATBE-Synthese, die Raffinatwäsche oder die Crack-C4-Hydrierung. In beiden Fällen werden Alkohole (ATBE-Synthese) oder polare Verunreinigungen (Raffinatwäsche, Crack-C4-Hydrierung) mit einer Wasserwäsche entfernt.

Der im erfindungsgemäßen Verfahren gewaschene Gasstrom, aus dem die organischen Bestandteile entfernt werden, ist insbesondere mit organischen Bestandteilen beladene Luft oder Stickstoff, das als Abgas anfällt. Solche Abgasströme aus Luft oder Stickstoff kommen in der chemischen Industrie an vielen Stellen vor. In einer bevorzugten Ausführungsform fällt der Gasstrom aus Luft oder Stickstoff bei der Lagerung oder Abfüllung an. Insbesondere stammt der Gasstrom aus Luft oder Stickstoff aus zumindest nicht vollständig flüssig gefüllten Behältern und fällt durch Verdrängung bei der Abfüllung an oder stammt aus Tanks im Füllzustand, wo er im Rahmen der Tankbeatmung durch Verdrängung des Gaspolsters von Tanks und Behältern anfällt.

Die organischen Bestandteile, die im Verfahren aus dem Gasstrom gewaschen werden und daher in dem Gasstrom enthalten sind, können eine Vielzahl von organischen Stoffen aus der chemischen Industrie sein. Beispiele für organische Bestandteile sind MTBE, ETBE, Methanol, Ethanol, C6- bis C16-Kohlenwasserstoffe, wie Diisobuten, Triisobuten, Di-n-Buten, Dodecen, Tetradecen, Tetradecan, Hexan, Isooctan, 1-Octen oder Decen, oder Mischungen daraus. Die im zu reinigenden Gasstrom enthaltenden Mengen entsprechen vorzugsweise ihrem Dampfdruck bei der entsprechenden Lager- bzw. Abfülltemperatur, ggf. der Umgebungstemperatur.

Der organische Bestandteile enthaltende Gasstrom wird im erfindungsgemäßen Verfahren in einer ersten Wascheinheit mit einem flüssigen alkoholischen Waschmittel in Kontakt gebracht und dadurch gewaschen. Das heißt die organischen Bestandteile des zu reinigen Gasstroms gehen zumindest teilweise in das Waschmittel über. Es entsteht ein an den organischen Bestandteilen reduzierter Gasstrom, der nun einen Teil des alkoholischen Waschmittels als alkoholische Bestandteile enthält, die durch das Waschen in den Gasstrom gelangen, und ein mit organischen Bestandteilen beladenes alkoholisches Waschmittel.

Die erste Wascheinheit ist vorzugsweise eine geeignete Kolonne, bei der sichergestellt wird, dass Gasphase (der zu waschende Gasstrom) und die Flüssigphase (das alkoholische Waschmittel) ausreichend Kontakt miteinander haben. Dazu eignet sich insbesondere eine Kolonne, die eine Kontaktzone, umfassend eine oder mehrere Packungen oder einen oder mehrere Ringschüttungen, aufweist. Die in der ersten Wascheinheit eingesetzte Kolonne wird vorzugsweise im leichten Unterdruck bei maximal - 30 mbarü (mbarü = Millibar Überdruck) betrieben. Ein solcher Unterdruck kann durch eine geeignete Pumpe erzeugt werden, beispielsweise eine Wasserstrahlpumpe. Die Pumpe ist vorzugsweise hinter der Kolonne, also zwischen erster und zweiter Wascheinheit, angeordnet. Der in die Kolonne geleitete Gasstrom kann dann durch den erzeugten Unterdruck zum und aus dem Kopf der Kolonne gezogen werden.

Der Zulauf des Gasstroms zur Kolonne der ersten Wascheinheit ist vorzugsweise oberhalb des Sumpfstandes der Kolonne angeordnet, aber unterhalb der Kontaktzone. Der Zulauf für das flüssige alkoholische Waschmittel zur Kolonne der ersten Wascheinheit ist dagegen vorzugsweise oberhalb des Zulaufs des Gasstroms und oberhalb der Kontaktzone angeordnet. Dadurch können der zu waschende Gasstrom und das flüssige alkoholische Waschmittel im Gegenstrom in der Kontaktzone miteinander in Kontakt gebracht werden, was im vorliegenden Fall für eine gute Waschleistung sorgt.

Die Temperatur der ersten Wascheinheit, insbesondere in der Kolonne der ersten Wascheinheit, ist grundsätzlich variabel. In einer bevorzugten Ausführungsform entspricht die Temperatur in der ersten Wascheinheit der jeweiligen Umgebungstemperatur.

Das alkoholische Waschmittel sollte in der ersten Wascheinheit in gewissen Mengen eingesetzt werden, um eine ausreichende Waschwirkung zu erzielen. Das sind erfindungsgemäß wobei pro m³ zu reinigendem Gasstrom mindestens 0,7 kg alkoholisches Waschmittel, vorzugsweise mindestens 1,2 kg alkoholisches Waschmittel und pro kg der zu entfernenden organischen Bestandteile mindestens 4 kg alkoholisches Waschmittel, vorzugsweise mindestens 8 kg alkoholisches Waschmittel. In einer besonders bevorzugten Ausführungsform werden pro m³ zu reinigendem Gasstrom mindestens 1,5 kg alkoholisches Waschmittel, weiterhin bevorzugt mindestens 5 kg alkoholisches Waschmittel, weiterhin bevorzugt mindestens 7 kg alkoholisches Waschmittel, besonders bevorzugt mindestens 8 kg alkoholisches Waschmittel eingesetzt. In einer ebenso bevorzugten Ausführungsform werden pro kg der zu entfernenden organischen Bestandteile mindestens 11 kg alkoholisches Waschmittel, weiterhin bevorzugt mindestens 15,5 kg alkoholisches Waschmittel, besonders bevorzugt mindestens 17 kg alkoholisches Waschmittel eingesetzt.

Dadurch werden die organischen Bestandteile des zu reinigen Gasstroms insbesondere zu über 90%, weiterhin bevorzugt zu über 95%, weiterhin bevorzugt zu über 98%, besonders bevorzugt zu über 99% aus dem zu reinigenden Gasstrom entfernt.

Das alkoholische Waschmittel, welches in der ersten Wascheinheit eingesetzt wird, ist insbesondere ein Alkohol, der bei -10 °C flüssig ist. Bevorzugt ist das flüssige alkoholische Waschmittel ein C1- bis C4-Alkohol, besonders bevorzugt ist Methanol oder Ethanol. Methanol oder Ethanol sind auch deshalb bevorzugt, weil es anlagen- und prozesstechnisch relativ einfach in einen chemischen Verbund der Petrochemie zu integrieren ist. Ist das flüssige alkoholische Waschmittel Methanol, kann das mit den organischen Bestandteilen aus dem Gasstrom beladene Methanol erfindungsgemäß zur Herstellung von MTBE eingesetzt werden. Ist das flüssige alkoholische Waschmittel Ethanol, kann das mit den organischen Bestandteilen aus dem Gasstrom beladene Ethanol erfindungsgemäß zur Herstellung von ETBE eingesetzt werden. Die organischen Bestandteile, die aus dem Gasstrom entfernt worden sind, werden dann über die MTBE- bzw. ETBE-Herstellung ausgeschleust. Das mit organischen Bestandteilen beladene Waschmittel enthält maximal 10 Gew.-% Organik, bevorzugt < 8 Gew.-%, besonders bevorzugt < 5 Gew.-%. Solche Mengen sind z.B. bei der MTBE-Synthese oder ETBE-Synthese unkritisch.

Der die alkoholischen Bestandteile enthaltende Gasstrom aus der ersten Wascheinheit wird im erfindungsgemäßen Verfahren in einer zweiten Wascheinheit mit einem flüssigen wässrigen Waschmittel in Kontakt gebracht und dadurch gewaschen. Das heißt die alkoholischen Bestandteile des zu reinigen Gasstroms gehen zumindest teilweise in das Waschmittel über. Die alkoholischen Bestandteile, die im zweiten Waschschritt entfernt werden, bestehen zum überwiegenden Teil aus dem alkoholischen Waschmittel des ersten Waschschritts bzw. der ersten Wascheinheit. Beim Waschen in der zweiten Wascheinheit entsteht ein an organischen und alkoholischen Bestandteilen reduzierter Gasstrom und ein mit alkoholischen Bestandteilen beladenes wässriges Waschmittel. Das mit alkoholischen Bestandteilen beladene wässrige Waschmittel kann z.B. zu einer Extraktion bzw. einem Waschen innerhalb einer MTBE-Synthese geleitet werden, um dort Methanol aus den Raffinatströmen auszuwaschen.

Die zweite Wascheinheit ist vorzugsweise eine geeignete Kolonne, bei der sichergestellt wird, dass die Gasphase (der zu waschende Gasstrom) und die Flüssigphase (das wässrige Waschmittel) in einer zweiten Kontaktzone ausreichend Kontakt haben, um die alkoholischen Bestandteile auszuwaschen. Dazu eignet sich insbesondere eine Kolonne, die in der zweiten Kontaktzone eine oder mehrere Packungen oder einen oder mehrere Ringschüttungen aufweisen. Die in der zweiten Wascheinheit eingesetzte Kolonne wird vorzugsweise im beim jeweiligen Umgebungsdruck betrieben. Besondere Aufbauten sind daher nicht erforderlich, könnten aber eingesetzt werden.

Der Zulauf des Gasstroms zur Kolonne der zweiten Wascheinheit ist vorzugsweise oberhalb über des Sumpfstandes der Kolonne, aber unterhalb der zweiten Kontaktzone angeordnet. Der Zulauf für das flüssige wässrige Waschmittel zur Kolonne der zweiten Wascheinheit ist dagegen vorzugsweise oberhalb des Zulaufs des Gasstroms und oberhalb der zweiten Kontaktzone angeordnet. Dadurch können der zu waschende Gasstrom und das flüssige wässrige Waschmittel im Gegenstrom in der zweiten Kontaktzone miteinander in Kontakt gebracht werden, was im vorliegenden Fall für eine gute Waschleistung sorgt.

Die Temperatur der zweiten Wascheinheit, insbesondere in der Kolonne der zweiten Wascheinheit, ist grundsätzlich variabel. In einer bevorzugten Ausführungsform entspricht die Temperatur in der zweiten Wascheinheit der jeweiligen Umgebungstemperatur.

Das wässrige Waschmittel sollte in der zweiten Wascheinheit in gewissen Mengen eingesetzt werden, um eine ausreichende Waschwirkung zu erzielen. Das sind erfindungsgemäß wobei pro m3 zu reinigendem Gasstrom mindestens 0,7 kg wässriges Waschmittel, vorzugsweise mindestens 1,2 kg wässriges Waschmittel und pro kg der zu entfernenden organischen Bestandteile mindestens 4 kg wässriges Waschmittel, vorzugsweise mindestens 8 kg wässriges Waschmittel. In einer besonders bevorzugten Ausführungsform werden pro m³ zu reinigendem Gasstrom mindestens 1,3 kg wässriges Waschmittel, besonders bevorzugt mindestens 1,5 kg wässriges Waschmittel eingesetzt. In einer ebenso bevorzugten Ausführungsform werden pro kg der zu entfernenden organischen Bestandteile mindestens 9,5 kg wässriges Waschmittel, besonders bevorzugt mindestens 10,5 kg wässriges Waschmittel eingesetzt.

Dadurch werden die alkoholischen Bestandteile des zu reinigen Gasstroms insbesondere zu über 90%, weiterhin bevorzugt zu über 95%, weiterhin bevorzugt zu über 98%, besonders bevorzugt zu über 99% aus dem zu reinigenden Gasstrom entfernt.

Das wässrige Waschmittel, welches in der zweiten Wascheinheit eingesetzt wird, kann grundsätzliche fast jede wässrige Lösung sein. Bevorzugt wird als wässriges Waschmittel in der zweiten Wascheinheit aber Wasser eingesetzt. Das Wasser hat insbesondere eine Reinheit von mindestens 99,9%. Sollte Methanol als alkoholisches Waschmittel eingesetzt werden, empfiehlt es sich, dass das eingesetzte Wasser maximal 1000 Gew.-ppm, bevorzugt maximal 500 Gew.-ppm, weiter bevorzugt maximal 200 Gew.-ppm, besonders bevorzugt maximal 80 Gew.-ppm an Methanol enthält. Nach dem Einsatz in der zweiten Wascheinheit kann das Wasser maximal 30 Gew.-%, bevorzugt < 25 Gew.-%, besonders bevorzugt < 20 Gew.-% Alkohol enthalten.

Der aus dem erfindungsgemäßen Verfahren erhaltene an organischen und alkoholischen Bestandteilen reduzierte Gasstrom enthält insbesondere nicht mehr als 600 mg organische und alkoholische Bestandteile pro m³ Gas, bevorzugt nicht mehr als 400 mg organische und alkoholische Bestandteile pro m³ Gas, besonders bevorzugt nicht mehr als 200 mg organische und alkoholische Bestandteile pro m³ Gas.

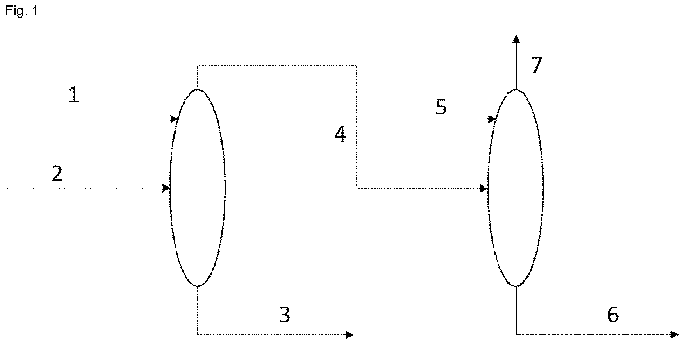

Fig. 1 ist eine schematische Darstellung des erfindungsgemäßen Verfahrens. Nicht dargestellt sind beispielsweise zugehörige Aggregate wie Pumpen, Wärmetauscher o. ä. Der zu reinigende Gasstrom (2) wird zur ersten Wascheinheit geführt, wo oberhalb des Zulaufs des zu reinigenden Gasstroms (2) das alkoholische Waschmittel (1) zugeführt wird. Dadurch wird das alkoholische Waschmittel (1) mit dem zu reinigenden Gasstrom (2) in Kontakt gebracht. Die organischen Bestandteile gehen dabei weitestgehend in das alkoholische Waschmittel (1) über. Das mit den organischen Bestandteilen beladene alkoholische Waschmittel (3) wird dann über den Sumpf aus der ersten Wascheinheit abgenommen. Der an den organischen Bestandteilen reduzierte Gasstrom (4), der ein Teil des alkoholischen Waschmittels als alkoholische Bestandteile enthält wird dann zur zweiten Wascheinheit geführt. Dort wird es mit dem wässrigen Waschmittel (5), welches oberhalb des Gasstroms (4) zugeführt wird, in Kontakt gebracht. Dadurch werden die alkoholischen Bestandteile aus dem Gasstrom weitestgehend entfernt. Es fällt ein an organischen und alkoholischen Bestandteilen reduzierter Gasstrom (7) am Kopf der zweiten Wascheinheit und ein mit alkoholischen Bestandteilen beladenes wässriges Waschmittel (6) im Sumpf der zweiten Wascheinheit an.

### Beispiele:

### Beispiel 1(nicht erfindungsgemäß):

In einer zweistufigen Wäsche wird in der ersten Wascheinheit unterhalb der Kontaktzone (Packung Sulzer M250.Y; Packungsbett: 5,9m Höhe und 200mm Durchmesser) ein 30°C warmer Gasstrom von 85 m³/h bestehend aus N₂ und 39,6 kg/h gasförmigen MTBE eingeleitet, während oberhalb der Kontaktzone 20°C warmes, flüssiges MeOH mit 50 kg/h eingeführt wird. Der am Kopf dieser ersten Waschkolonne abgenommene Gasstrom enthält nun 13 kg/h gasförmiges MeOH und wird in die zweite Wascheinheit geleitet, wo er unterhalb der dortigen Kontaktzone (Packung r Sulzer M250.Y; Packungsbett: 5,9m Höhe und 200mm Durchmesser) eingeblasen wird. Oberhalb dieser Kontaktzone wird 25°C warmes, flüssiges Wasser mit 20 kg/h eingeführt. Am Kopf dieser zweiten Waschkolonne wird das verbleibende Abgas abgeführt, das aufgrund der Mischungswärme von MeOH/Wasser 40°C warm ist. Es enthält bei 87 m3/h Gasstrom etwa 4,7 kg/h MTBE und 2 kg/h MeOH. Die Abtrennrate liegt damit bei 88,1% für MTBE und 84,6% für MeOH. Der mit MTBE beladene MeOH-Waschstrom wird dem MeOH-Einsatzstrom einer MTBE-Synthese zugeführt. Das mit MeOH und MTBE beladene Waschwasser wird einem Wasserstrom zugeführt, der in einer C4/Wasserwäsche der MTBE-Syntheseanlage eingesetzt wird.

### Beispiel 2 (nicht erfindungsgemäß):

In einer zweistufigen Wäsche wird in der ersten Wascheinheit unterhalb der Kontaktzone (Packung Sulzer M250.Y; Packungsbett: 5,9m Höhe und 200mm Durchmesser) ein 30°C warmer Gasstrom von 85 m³/h bestehend aus N₂ und 39,6 kg/h gasförmigen MTBE eingeleitet, während oberhalb der Kontaktzone 20°C warmes, flüssiges MeOH mit 50 kg/h eingeführt wird. Der am Kopf dieser ersten Waschkolonne abgenommene Gasstrom enthält nun 13 kg/h gasförmiges MeOH und wird in die zweite Wascheinheit geleitet, wo er unterhalb der dortigen Kontaktzone (Packung Sulzer M250.Y; Packungsbett: 5,9m Höhe und 200mm Durchmesser) eingeblasen wird. Oberhalb dieser Kontaktzone wird 25°C warmes, flüssiges Wasser mit 150 kg/h eingeführt. Am Kopf dieser zweiten Waschkolonne wird das verbleibende Abgas abgeführt, das aufgrund der Mischungswärme von MeOH/Wasser 26,5°C warm ist. Es enthält bei 78 m³/h Gasstrom etwa 4,7 kg/h MTBE und 0,001 kg/h MeOH. Die Abtrennrate liegt damit bei 88,1% für MTBE und >99,9% für MeOH. Der mit MTBE beladene MeOH-Waschstrom wird dem MeOH-Einsatzstrom einer MTBE-Synthese zugeführt. Das mit MeOH und MTBE beladene Waschwasser wird einem Wasserstrom zugeführt, der in einer C4/Wasserwäsche der MTBE-Syntheseanlage eingesetzt wird.

### Beispiel 3 (nicht erfindungsgemäß):

In einer zweistufigen Wäsche wird in der ersten Wascheinheit unterhalb der Kontaktzone (Packung Sulzer M250.Y; Packungsbett: 5,9m Höhe und 200mm Durchmesser) ein 30°C warmer Gasstrom von 85 m³/h bestehend aus N₂ und 39,6 kg/h gasförmigen MTBE eingeleitet, während oberhalb der Kontaktzone 20°C warmes, flüssiges MeOH mit 750 kg/h eingeführt wird. Der am Kopf dieser ersten Waschkolonne abgenommene Gasstrom enthält nun 16,4 kg/h gasförmiges MeOH und wird in die zweite Wascheinheit geleitet, wo er unterhalb der dortigen Kontaktzone (Packung Sulzer M250.Y; Packungsbett: 5,9m Höhe und 200mm Durchmesser) eingeblasen wird. Oberhalb dieser Kontaktzone wird 25°C warmes, flüssiges Wasser mit 20 kg/h eingeführt. Am Kopf dieser zweiten Waschkolonne wird das verbleibende Abgas abgeführt, das aufgrund der Mischungswärme von MeOH/Wasser 43°C warm ist. Es enthält bei 92 m3/h Gasstrom etwa 0,004 kg/h MTBE und 3,4 kg/h MeOH. Die Abtrennrate liegt damit bei >99,9% für MTBE und 79,3% für MeOH. Der mit MTBE beladene MeOH-Waschstrom wird dem MeOH-Einsatzstrom einer MTBE-Synthese zugeführt. Das mit MeOH und MTBE beladene Waschwasser wird einem Wasserstrom zugeführt, der in einer C4/Wasserwäsche der MTBE-Syntheseanlage eingesetzt wird.

### Beispiel 4 (erfindungsgemäß):

In einer zweistufigen Wäsche wird in der ersten Wascheinheit unterhalb der Kontaktzone (Packung Sulzer M250.Y; Packungsbett: 5,9m Höhe und 200mm Durchmesser) ein 30°C warmer Gasstrom von 85 m3/h bestehend aus N₂ und 39,6 kg/h gasförmigen MTBE eingeleitet, während oberhalb der Kontaktzone 20°C warmes, flüssiges MeOH mit 750 kg/h eingeführt wird. Der am Kopf dieser ersten Waschkolonne abgenommene Gasstrom enthält nun 16,4 kg/h gasförmiges MeOH und wird in die zweite Wascheinheit geleitet, wo er unterhalb der dortigen Kontaktzone (Packung Sulzer M250.Y; Packungsbett: 5,9m Höhe und 200mm Durchmesser) eingeblasen wird. Oberhalb dieser Kontaktzone wird 25°C warmes, flüssiges Wasser mit 150 kg/h eingeführt. Am Kopf dieser zweiten Waschkolonne wird das verbleibende Abgas abgeführt, das aufgrund der Mischungswärme von MeOH/Wasser 31 °C warm ist. Es enthält bei 86 m3/h Gasstrom etwa 0,004 kg/h MTBE und 0,01 kg/h MeOH. Die Abtrennrate liegt damit bei >99,9% für MTBE und >99,9% für MeOH. Die Summe aus MeOH und MTBE im Abgas beträgt damit erfindungsgemäß nur 14 g/h. Der mit MTBE beladene MeOH-Waschstrom wird dem MeOH-Einsatzstrom einer MTBE-Synthese zugeführt. Das mit MeOH und MTBE beladene Waschwasser wird einem Wasserstrom zugeführt, der in einer C4/Wasserwäsche der MTBE-Syntheseanlage eingesetzt wird.

## Patentansprüche

1. Verfahren zur Entfernung von organischen Bestandteilen aus einem Gasstrom in einer zweistufigen Wäsche, wobei
der Gasstrom in einer ersten Wascheinheit mit einem flüssigen alkoholischen Waschmittel gewaschen wird, wodurch die organischen Bestandteile des zu reinigen Gasstroms zumindest teilweise in das Waschmittel übergehen und wodurch ein an organischen Bestandteilen reduzierter Gasstrom, der einen Teil des alkoholischen Waschmittels als alkoholische Bestandteile enthält, und ein mit organischen Bestandteilen beladenes alkoholisches Waschmittel erhalten wird; und
der an organischen Bestandteilen reduzierte Gasstrom in einer zweiten Wascheinheit mit einem wässrigen Waschmittel gewaschen wird, wodurch die alkoholischen Bestandteile zumindest teilweise in das Wachmittel übergehen und wodurch ein an organischen und alkoholischen Bestandteilen reduzierter Gasstrom und ein mit alkoholischen Bestandteilen beladenes wässriges Waschmittel erhalten wird;
**dadurch gekennzeichnet, dass** in beiden Wascheinheiten jeweils pro m³ zu reinigendem Gasstrom mindestens 0,7 kg Waschmittel und pro kg der zu entfernenden organischen Bestandteile mindestens 4 kg Waschmittel eingesetzt werden, und dass
das mit organischen Bestandteilen beladene alkoholische Waschmittel in ein (chemisches) Verfahren (zurück)geführt wird, in dem der Alkohol als Einsatzstoff verwendet wird, und das mit alkoholischen Bestandteilen beladene wässrige Waschmittel in ein (chemisches) Verfahren zurückgeführt wird, in dem das Wasser als Einsatzstoff oder Extraktionsmittel verwendet wird.

2. Verfahren nach Anspruch 1, wobei die organischen Bestandteile des zu reinigen Gasstroms in der ersten Wascheinheit zu über 90%, bevorzugt zu über 95%, weiterhin bevorzugt zu über 98%, besonders bevorzugt zu über 99% in das Waschmittel übergehen.

3. Verfahren nach Anspruch 1 oder 2, wobei die alkoholischen Bestandteile in der zweiten Wascheinheit zu über 90%, bevorzugt zu über 95%, weiterhin bevorzugt zu über 98%, besonders bevorzugt zu über 99% in das Wachmittel übergehen.

4. Verfahren nach einem der vorhergehenden Ansprüche, wobei das (chemische) Verfahren, in das das Wasser (zurück)geführt wird, eine TBA-Synthese, eine Hydrolyse oder eine Hydroxycarbonylierung ist, bei der das Wasser Edukt ist, oder das Wasser in einem (chemischen) Verfahren als Wasch- oder Extraktionsmittel eingesetzt wird, beispielsweise in der ATBE-Synthese, der Raffinatwäsche oder der Crack-C4-Hydrierung.

5. Verfahren nach einem der vorhergehenden Ansprüche, wobei das (chemische) Verfahren, in das der Alkohol (zurück)geführt wird, eine Reaktion ist, bei der Methanol als Edukt eingesetzt wird, beispielsweise eine Veretherung, eine Methoxycarbonylierung oder eine Veresterung.

6. Verfahren nach einem der vorhergehenden Ansprüche, wobei der Gasstrom mit organischen Bestandteilen beladene Luft oder Stickstoff aus Behältern ist, die durch Verdrängung bei der Abfüllung oder durch Verdrängung des Gaspolsters von Tanks und Behältern entstehen.

7. Verfahren nach einem der vorhergehenden Ansprüche, wobei die erste Wascheinheit im leichten Unterdruck bei maximal - 30 mbarü betrieben wird.

8. Verfahren nach einem der vorhergehenden Ansprüche, wobei das flüssige alkoholische Waschmittel ein Alkohol ist, der bei -10 °C flüssig ist.

9. Verfahren nach Anspruch 8, wobei das das flüssige alkoholische Waschmittel ein C1- bis C4-Alkohol, vorzugsweise Methanol oder Ethanol ist.

10. Verfahren nach Anspruch 9, wobei das das flüssige alkoholische Waschmittel Methanol oder Ethanol ist und das beladene Methanol bzw. Ethanol aus dem Verfahren zur Herstellung von MTBE bzw. ETBE eingesetzt wird, wodurch die organischen Bestandteile über die MTBE/ETBE-Herstellung ausgeschleust werden.

11. Verfahren nach einem der vorherigen Ansprüche, wobei die zweite Wascheinheit bei Umgebungsdruck betrieben wird.

12. Verfahren nach einem der vorherigen Ansprüche, wobei ein mit alkoholischen Bestandteilen beladenes wässriges Waschmittel entsteht, dass zu einer Extraktion einer MTBE oder ETBE-Synthese geleitet wird, um dort Methanol bzw. Ethanol aus den Raffinatströmen auszuwaschen.

13. Verfahren nach einem der vorherigen Ansprüche, wobei das eingesetzte Wasser maximal 1000 Gew.-ppm, bevorzugt maximal 500 Gew.-ppm, weiter bevorzugt maximal 200 Gew.-ppm, besonders bevorzugt maximal 80 Gew.-ppm an Methanol enthält.

14. Verfahren nach einem der vorhergehenden Ansprüche, wobei die alkoholischen Bestandteile, die im zweiten Waschschritt entfernt werden, zum überwiegenden Teil aus dem alkoholischen Waschmittel des ersten Waschschritts bestehen.

15. Verfahren nach einem der vorhergehenden Ansprüche, wobei der an organischen und alkoholischen Bestandteilen reduzierte Gasstrom nicht mehr als 300 mg organische und alkoholische Bestandteile pro m³ Gas, bevorzugt nicht mehr als 200 mg organische und alkoholische Bestandteile pro m³ Gas, besonders bevorzugt nicht mehr als 100 mg organische und alkoholische Bestandteile pro m³ Gas enthält.
